# EUROPEAN PATENT SPECIFICATION

(11) **EP 0 816 677 B1**
(45) Date of publication and mention of the grant of the patent: **24.09.2003**
(21) Application number: 97304881.2
(22) Date of filing: 03.07.1997
(51) Int. Cl.: F04B 11/00

(54) **Reciprocating pump**
Hubkolbenpumpe
Pompe à piston alternatif

(30) Priority: 03.07.1996 KR 9626856
(43) Date of publication of application: 07.01.1998
(73) Proprietor: Kim, Seong-Cheol, Seoul (KR)
(72) Inventor: Kim, Seong-Cheol, Seoul (KR)
(74) Representative: Tunstall, Christopher Stephen

(56) References cited:
- DE-C- 549 790
- DE-C- 802 903
- FR-A- 1 053 207
- US-A- 1 445 035
- US-A- 3 447 557

## Description

The present invention relates to pump which includes a reciprocating pumping member for varying the volume of a pumping chamber, such as a diaphragm pump.

Various types of pumps are known to those skilled in the art. For example FR 1053207, DE 549790, US 1,445,035 and DE 802903 all disclose pumps of one kind or another.

A typical diaphragm pump pumps fluid (liquid) by converting rotational movement of a motor into a reciprocating movement by means of a device such as a cam. Since the amount of the discharged fluid from the diaphragm pump fluctuates by very a small volume, the diaphragm pump is generally used for precisely dispensing of liquid chemicals or medicines.

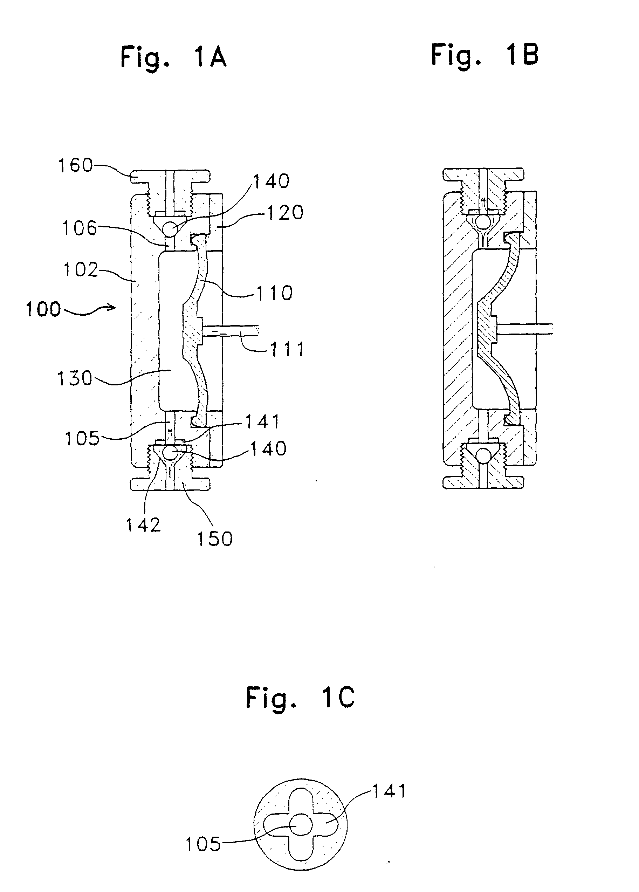

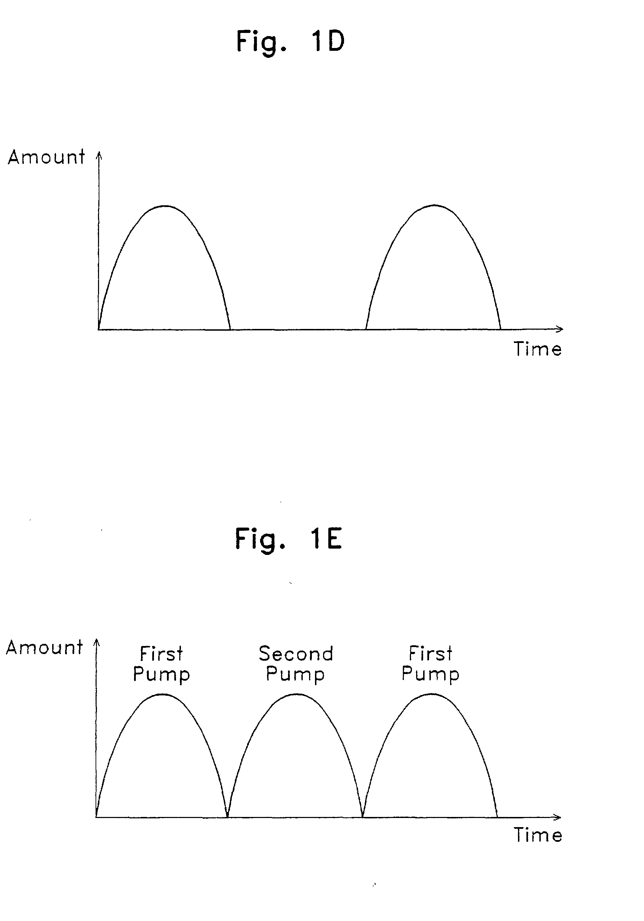

FIGs. 1A to 1E are cross sectional views illustrating one conventional diaphragm pump. As shown in FIG. 1A, a diaphragm 110 is supported by a support ring 120 to form a pump chamber 130 in an open area of a pump head 100. A suction hole 105 and a discharge hole 106 are located at the upper portion and the lower portion of a body 102, respectively.

The suction hole 105 and the discharge hole 106 are opened/closed by a check ball 140. Referring to FIG. 1C, an opening-side valve seat 141 having a cross-shaped groove is formed at the suction end of the suction hole 105 of the pump head 100 and at one end of a discharge connector 160. A closing-side valve seat 142 having a tapered shape is formed at one end of a suction connector 150 and the discharge side of the discharge hole 106 of the pump head 100.

The following describes the operation of the diaphragm pump constructed in such a manner as mentioned above. When a motor (not illustrated) is driven to operate the pump, the rotational movement of the motor is changed to reciprocating movement of a diaphragm shaft 111 by a device such as an eccentric cam, and thus the diaphragm 110 is driven back and forth. FIG. 1A illustrates the diaphragm being driven back, that is, a suction process, and FIG. 1B illustrates the diaphragm being driven forward, that is, a discharge process.

During the suction process, due to the internal pressure, each check ball 140 of the suction side and the discharge side moves toward the centre of the pump head 100. Subsequently, since the opening-side valve seat 141 is formed at the end of the suction hole 105 of the pump head 100, fluid is drawn into the pump chamber 130 through the cross-shaped groove. The discharge hole 106 is closed by the check ball 140. On the other hand, during the discharge process, each check ball 140 moves away from the pump head 100 to the outside such that the suction hole 105 is closed and only the discharge hole 106 is opened.

Subsequently, the fluid in the pump chamber 130 is discharged through the cross-shaped groove formed at the discharge-side connector 160.

The advantage of such a conventional diaphragm pump in the long term is that the average amount of the discharged fluid is very uniform. However, since the pumping operation is separated into suction and discharge processes, and it is intermittently performed, there is a fundamental problem of pulsation of the discharged fluid, as shown in FIG. 1D.

To prevent such pulsation of the discharged fluid, two or more pumps can be connected in parallel and operated in different strokes, as shown in FIG. 1E.

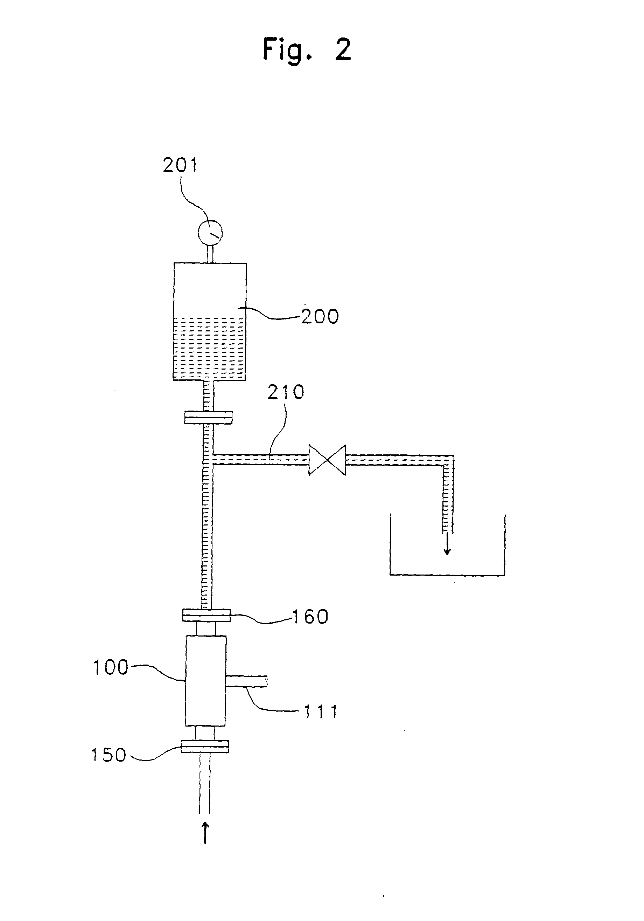

Referring to FIG. 2, an alternative is explained. By use of an air chamber 200 in a fluid pipe, during the discharge process, the air in the air chamber 200 can be pressurized, and the amount of fluid discharging from a discharge pipe 210 is reduced. During the suction process, due to the expansion of the pressurized air, the fluid stored in the air chamber 200 during the discharge process is discharged through the discharge pipe 210. For reference, the unmentioned reference numeral 201 is a pressure gauge.

However, the benefit of using two pumps connected in parallel is significantly offset by the substantial increase in installation cost. Moreover, in the case of installing the air chamber in the fluid pipe, it is difficult to perform the installation operation. Also, it is impractical to us an air chamber when the connector of the pump or the fluid pipe passage is a tube. Furthermore, damage to the connector caused by the vibration of the air chamber when operating the pump frequently occur. Furthermore, since such methods cannot fundamentally remove the pulsation of the discharged fluid, water hammer caused by the pulsation can be generated and it may destroy pipes, especially at high pressure.

These problems are common to pumps which use a reciprocating pumping member to vary the volume of a pumping chamber. Therefore, it is an objective of the present invention to provide a reciprocating pump having a simple structure for efficiently preventing the pulsation of a discharged fluid.

According to a first aspect of the present invention there is provided a fluid pump comprising a housing defining a pumping chamber, an outlet for discharge of fluid from the pumping chamber and a reciprocating pumping member for varying the volume of the pumping chamber, in which the pumping chamber has an inlet for admission of fluid to be pumped, said housing also defines a buffer chamber, separated from said pumping chamber by a common wall, into which fluid is discharged from the outlet of said pumping chamber, whereby the buffer chamber has an outlet for discharge of fluid and at least part of the buffer chamber contains a gas which is compressed as fluid is admitted into the buffer chamber and expands as fluid is discharged from the buffer chamber; characterised in that said pump further comprises a partitioning plate located within the buffer chamber to divide the buffer chamber into a part containing the gas and a part into which the outlet from the pumping chamber opens, the two parts being in communication via an opening in the lower portion of the partitioning plate.

The outlet for discharge of fluid from the pumping chamber to the buffer chamber may be an aperture located in or adjacent to the common wall and the reciprocating pumping member may be a diaphragm.

The outlet from the buffer chamber may be formed in a discharge connector attached to the housing and may taper from a relatively large opening in the buffer chamber to a relatively small exit orifice.

The pump may further comprise a discharge pipe located within and connected to the outlet from the buffer chamber.

Preferably, the part of the buffer chamber containing the gas may be a central part and the part into which the outlet from the pumping chamber opens an outer, annular part.

Preferably, the housing comprises first and second bodies attached to one another and respectively defining at least in part the pumping and buffer chambers and the partitioning plate is integral with the second body and abuts the said common wall which is integral with the first body.

The present invention will now be described by way of example with reference to the accompanying drawings in which:
FIGs. 1A and 1B are cross sectional views showing the structure and operation of a conventional diaphragm pump;
FIG. 1C is a plan view of an opening-side value seat having a cross-shaped groove;
FIG. 1D is a graph showing the pulsation of the amount of discharged fluid;
FIG. 1E is a graph showing the pulsation of the amount of the discharged fluid in the case of reducing the pulsation effect;
FIG. 2 is a schematic view showing an air chamber being installed on a conventional fluid pipe passage;
FIG. 3A is a cross sectional view of a diaphragm pump according to a preferred embodiment of the present invention;
FIG. 3B is a front elevation view of an air chamber cut from the line B to B' of FIG. 3A;
FIGs. 4A and 4B are cross sectional views of another preferred embodiment of the present invention; and
FIGs. 5A to 5D are elevational views of yet further preferred embodiments of the present invention.

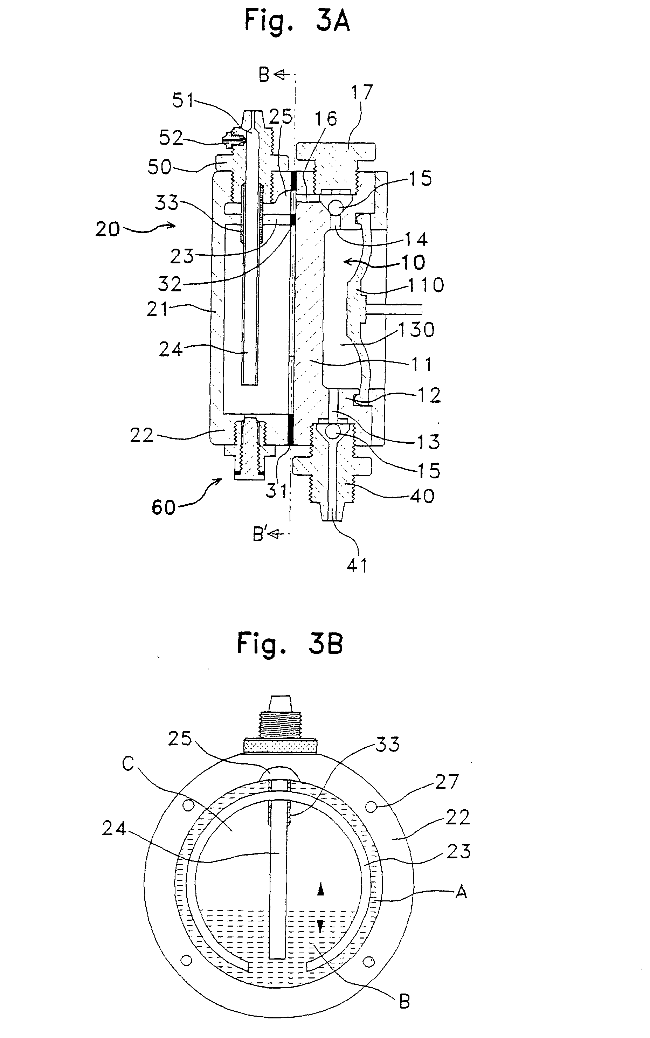

FIGs. 3A and 3B illustrate the structure and operation of a diaphragm pump according to one embodiment of the present invention. FIG. 3A is a cross sectional view of its main parts and FIG. 3B is a front elevational view of its air chamber.

According to the drawings, a suction hole 13 and a head-side discharge hole 14, which are closed and opened by a check ball 15, are respectively formed at the upper and lower portions of a body 12 of a pump head 10. A suction-side connector 40 having a suction passage 41 is attached to the outside of the suction hole 13. Moreover, a cap 17 is connected to the outside of the head-side discharge hole 14.

An air chamber 20 is installed on a side wall 11 of the pump head 10 such that the air chamber 20 is connected to the head-side discharge hole 14 via a connecting hole 16 formed at the side wall 11 of the pump head 10 and a connecting groove 25 formed at one side of a body 22 of the air chamber 20. However, the connecting groove 25 can be removed when the inner side wall of the body 22 is placed outside of the connecting hole 16 by increasing the internal diameter of the air chamber 20.

On the side wall 21 of the air chamber 20, a cylindrical partitioning plate 23, of which the lower portion is opened, is formed. A discharge-side connector 50 having a discharge-side discharge hole 51 is attached to the upper portion of the body 22. Consequently, the diameter of the exit side of the discharge hole 51 is smaller than that of the entrance side of the discharge hole 51. Although the lower portion of the partitioning plate 23 is exposed as it is open over its entire width, a circular shape is preferred for much simpler manufacturing.

At the lower portion of the discharge-side connector 50, a discharge pipe 24, which is pressure-fixed using packing 33 as an intermediary, penetrates the partitioning plate 23 extending to the lower portion of the air chamber.

Among the unmentioned reference numerals in the drawing, reference numerals 31 and 32 are packing for preventing the flow of air and fluid. Reference numeral 27 is a bolt hole for fixing the air chamber to the pump head. Finally, reference numerals 60 and 52 are a drain valve and an air vent valve, respectively.

The following describes the operation of the diaphragm pump formed as in the above embodiment. The state of the air chamber 20 in operation is shown in FIG. 3B. Specifically, the fluid to be pumped fills a space A and a space B. The space A is defined by the area in between the partitioning plate 23 and the body 22, and the space B is defined by the lower portion of the inside area of the partitioning plate 23. The air which fills a space C, which is defined by the upper portion of the inside surface of the partitioning plate 23, is isolated from the external environment by the packing 32 and 33 and the fluid which fills the lower portion, and the amount of air in the space C is always constant.

When the diaphragm 110 in the state shown in FIG. 3A moves in, that is, in the discharge process, the discharge-side check ball 15 moves up to open the head-side discharge hole 14. Simultaneously, the fluid previously drawn into a pump chamber 130 during a previous suction process is discharged to the inside of the air chamber 20 via the head-side discharge hole 14 and a connection hole 16. Subsequently, as the fluid pressure is increased inside the air chamber 20, the air in the space C is pressurized and thus the level of fluid inside the partitioning plate 23 rises. In other words, not all of the fluid expelled into the air chamber 20 via the connection pipe is discharged through the discharge pipe 24 and the discharge-side discharge hole 51. As a certain amount of fluid influences the level of fluid inside the partitioning plate 23 and the increasing air pressure, a sudden change in the amount of fluid being discharged can be prevented.

On the other hand, during the suction process, the head-side discharge hole 14 is closed to prevent back-flow of fluid inside of the air chamber 20, and fluid discharging to the air chamber 20 is stopped. However, due to the air pressure inside the air chamber 20 which is pressurized during the previous discharge process, the fluid inside the air chamber 20 is continuously discharged through the discharge pipe 24 and the discharge-side discharge hole 51, and the fluid level inside of the partitioning plate 23 gradually decreases. Accordingly, the amount of discharged fluid does not decrease rapidly, and it is almost equal to the amount of the fluid discharged during the discharge process.

Furthermore, since the diameter of the exit area of the discharge-side discharge hole 51 is smaller than that of the entrance area, even if there is a small change in the amount of fluid discharged through the discharge pipe 24, the amplitude of the change decreases as it passes through the small diameter exit area. Therefore, the pulsation effect of the discharged fluid is further reduced.

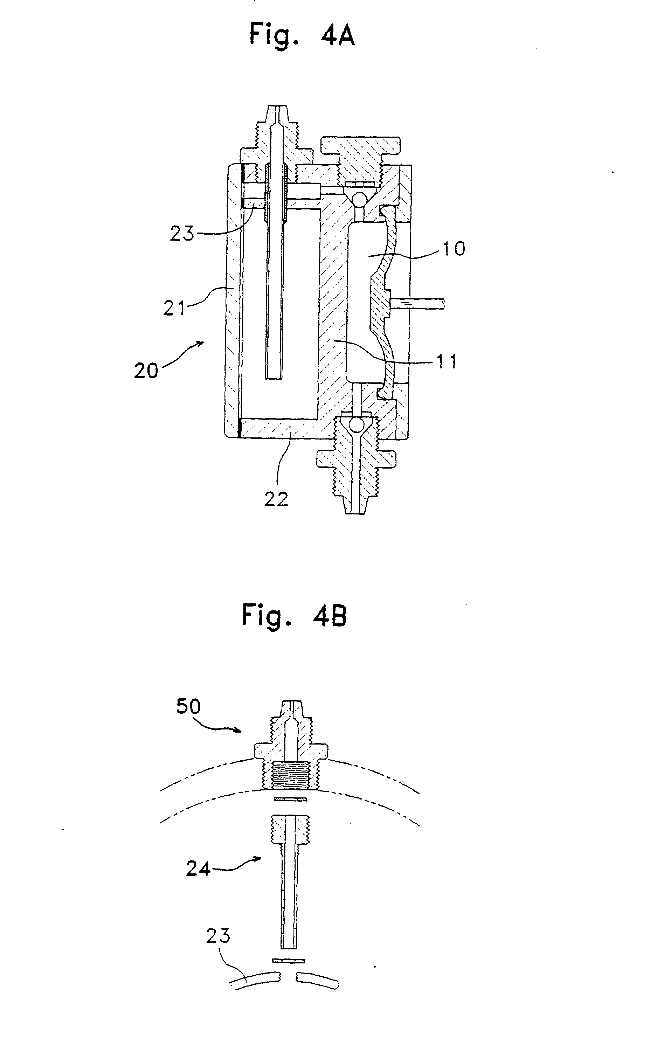

FIGs. 4A and 4B are sectional views showing another embodiment of the present invention. According to FIG. 4A, the partitioning plate 23 and the body 22 of the air chamber 20 are integrally formed at the side wall 11 of the pump head 10, and a separate side wall 21 is connected to the open area. The discharge pipe 24 is fixed to the discharge-side connector 50 and the partitioning plate 23 by means of screws, as illustrated in FIG. 4B.

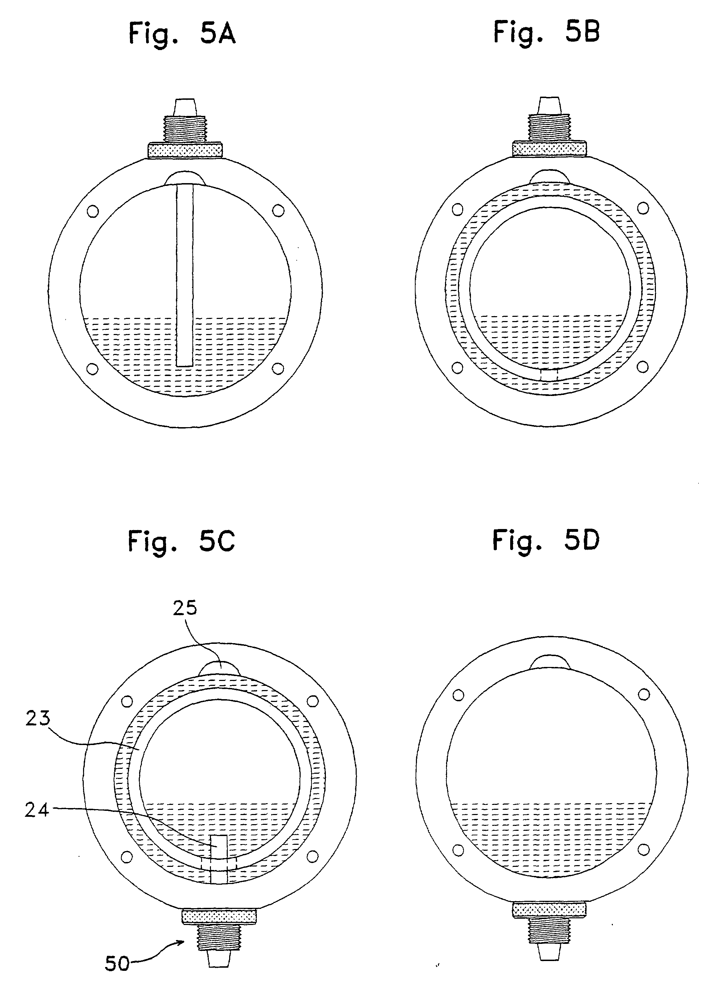

FIGS. 5A to 5D are sectional views illustrating further embodiments of the present invention. FIGs. 5A and 5B illustrate embodiments having the discharge-side discharge hole formed at the upper portion of the air chamber. Referring to FIG. 5A the partitioning plate is removed, and the fluid to be pumped which is in the lower portion of the air chamber is discharged to the discharge-side discharge hole of the upper portion of the air chamber through the discharge pipe.

Referring to FIG. 5B, in the case that the partitioning plate is installed, as the discharge-side discharge hole is in contact with the fluid to be pumped outside of the partitioning plate instead of the air, the discharge pipe can be removed. FIGs. 5C and 5D illustrate preferred embodiments showing the discharge-side discharge hole formed at the lower portion of the air chamber. Referring to FIG. 5C, the connecting groove 25 is formed in the upper portion of the air chamber, and the discharge-side connector 50 and the discharge-side discharge hole are formed at the lower portion of the air chamber, respectively. The partitioning plate 23 and the discharge pipe 24 are also provided.

Referring to FIG. 5D, in the case that the discharge-side discharge hole is formed at the lower portion of the air chamber, as the discharge-side discharge hole is directly connected with the fluid to be pumped without a separate partitioning plate, the partitioning plate and the discharge pipe can be removed.

The operating principles of the above-mentioned embodiments are similar to those of the embodiments of FIG. 3, and thus a detailed description of them will be omitted.

When the partitioning plate is removed as in the case of FIG. 5A or FIG. 5D, a small amount of air can slip back into the pump chamber through the connection hole during the closing action of the check ball according to the suction process. However, as the fluid is drawn through the suction hole during the suction process, even if a small amount of air escapes from the air chamber to the pump chamber, the air will be unable to slip through the suction hole. During the next discharge process, the air will be discharged back to the air chamber along with the fluid. Therefore, the amount of air loss in the air chamber caused by such a leakage is negligible.

As described above, the pump of the present invention proficiently prevents the pulsation effect of the discharged fluid. Moreover, since there is no need for installing extra parts, such as an air chamber and pipes, the installation of the pump becomes much easier, thereby reducing its installation cost.

Furthermore, even if the air chamber is installed to reduce the pulsation of fluid according to the conventional method, the fluid is still discharged intermittently from a diaphragm pump. As a result, the conventional diaphragm pump is noisy, and the pipes are frequently damaged due to water hammer. However, since the diaphragm pump of the present invention reduces the pulsation of the fluid with the pump itself before discharging the fluid, the pipes are guarded from such damage, and the level of noise can be reduced to an acceptable level.

Even though the present invention has been described in application to a certain type of a diaphragm pump for convenience, it should be noted that the present invention can be applied to any other type of diaphragm pumps or any other type of pumps in which pulsation of the fluid occurs by intermittent suction and discharge, i.e. in which a reciprocating pumping member varies the volume of a pumping chamber.

While the present invention has been described in connection with preferred embodiments, various modifications and equivalent arrangements can be made. For example, the connection hole can be replaced with a separate pipe, or the shape of the partitioning plate or the discharge-side connector can be modified.

## Claims

1. A fluid pump comprising a housing defining a pumping chamber. (10), an outlet (14) for discharge of fluid from the pumping chamber and a reciprocating pumping member (110) for varying the volume of the pumping chamber, in which the pumping chamber has an inlet (13) for admission of fluid to be pumped, wherein said housing also defines a buffer chamber, separated from said pumping chamber by a common wall, into which fluid is discharged from the outlet of said pumping chamber, whereby the buffer chamber has an outlet for discharge of fluid and at least part of the buffer chamber contains a gas which is compressed as fluid is admitted into the buffer chamber and expands as fluid is discharged from the buffer chamber; **characterised in that** said pump further comprises a partitioning plate (23) located within the buffer chamber to divide the buffer chamber into a part containing the gas (C) and a part (A) into which the outlet from the pumping chamber opens, the two parts being in communication via an opening in the lower portion (B) of the partitioning plate.

2. A pump according to claim 1 in which the outlet for discharge of fluid from the pumping chamber to the buffer chamber is an aperture located in or adjacent to the common wall.

3. A pump according to claim 1 or claim 2 in which the reciprocating pumping member is a diaphragm.

4. A pump according to any preceding claim in which the outlet from the buffer chamber is formed in a discharge connector attached to the housing.

5. A pump according to any preceding claim in which the outlet from the buffer chamber tapers from a relatively large opening in the buffer chamber to a relatively small exit orifice.

6. A pump according to any preceding claim further comprising a discharge pipe (24) located within and connected to the outlet from the buffer chamber.

7. A pump according to any preceding claim in which the part of the buffer chamber containing the gas is a central part and the part into which the outlet from the pumping chamber opens is an outer annular part.

8. A pump according to any preceding claim in which the housing comprises first and second bodies attached to one another and respectively defining at least in part the pumping and buffer chambers and the partitioning plate is integral with the second body and abuts the said common wall which is integral with the first body.

## Patentansprüche

1. Fluidpumpe, umfassend ein eine Pumpkammer (10) definierendes Gehäuse, einen Auslass (14) für den Ausfluss von Fluid aus der Pumpkammer und ein Kolbenpumpelement (110), um das Volumen der Pumpkammer zu variieren, wobei die Pumpkammer einen Einlass (13) für den Eintritt von zu pumpendem Fluid aufweist, **dadurch gekennzeichnet, dass** das genannte Gehäuse auch eine Pufferkammer definiert, die durch eine gemeinsame Wand von der genannten Pumpkammer getrennt ist, in die Fluid vom Auslass der genannten Pumpkammer ausfließt, wobei die Pufferkammer einen Auslass für den Ausfluss von Fluid hat und wenigstens ein Teil der Pufferkammer ein Gas enthält, das verdichtet wird, wenn Fluid in die Pufferkammer eintritt, und sich ausdehnt, wenn Fluid aus der Pufferkammer austritt; und wobei die genannte Pumpe ferner eine Trennplatte (23) umfasst, die sich in der Pufferkammer befindet, um die Pufferkammer in einen Teil, der das Gas (C) enthält, und einen Teil (A) unterteilt, in den der Auslass aus der Pumpkammer mündet, wobei die beiden Teile über eine Öffnung im unteren Abschnitt (B) der Trennplatte in Verbindung sind.

2. Pumpe nach Anspruch 1, bei der der Auslass für den Austritt von Fluid aus der Pumpkammer in die Pufferkammer ein Spalt in oder neben der gemeinsamen Wand ist.

3. Pumpe nach Anspruch 1 oder 2, bei der das Kolbenpumpelement eine Membran ist.

4. Pumpe nach einem der vorherigen Ansprüche, bei der der Auslass aus der Pufferkammer in einem an dem Gehäuse angebrachten Ausflussverbinder ausgebildet ist.

5. Pumpe nach einem der vorherigen Ansprüche, bei der sich der Auslass aus der Pufferkammer von einer relativ großen Öffnung in der Pufferkammer zu einer relativ kleinen Ausgangsmündung verjüngt.

6. Pumpe nach einem der vorherigen Ansprüche, ferner umfassend ein Ausflussrohr (24), das sich innerhalb des Auslasses aus der Pufferkammer befindet und mit diesem verbunden ist.

7. Pumpe nach Anspruch 7, bei der der das Gas enthaltende Teil der Pufferkammer und der Teil, in den der Auslass aus der Pumpkammer mündet, ein äußerer ringförmiger Teil ist.

8. Pumpe nach einem der vorherigen Ansprüche, bei der das Gehäuse einen ersten und einen zweiten Körper umfasst, die aneinander befestigt sind und jeweils wenigstens teilweise die Pump- und die Pufferkammer definieren, und wobei die Trennplatte mit dem zweiten Körper integral ist und an die genannte gemeinsame Wand anstößt, die mit dem ersten Körper integral ist.

## Revendications

1. Pompe à fluide comprenant un carter définissant une chambre de pompage (10), un orifice de sortie (14) servant à décharger du fluide à partir de la chambre de pompage et un élément de pompage alternatif (110) servant à faire varier le volume de la chambre de pompage, dans laquelle la chambre de pompage possède un orifice d'entrée (13) afin d'admettre du fluide destiné à être pompé, **caractérisée en ce que** ledit carter définit également une chambre intermédiaire, qui est séparée de ladite chambre de pompage par une paroi commune, dans laquelle du fluide est déchargé à partir de l'orifice de sortie de ladite chambre de pompage, cas dans lequel la chambre intermédiaire possède un orifice de sortie servant à décharger du fluide, et une partie au moins de la chambre intermédiaire contient un gaz qui est comprimé au fur et à mesure que du fluide est admis dans la chambre intermédiaire et se dilate au fur et à mesure que du fluide est déchargé à partir de la chambre intermédiaire ; et dans laquelle ladite pompe comprend en outre une plaque de séparation (23) qui est positionnée à l'intérieur de la chambre intermédiaire afin de diviser la chambre intermédiaire en une partie contenant le gaz (C) et une partie (A) dans laquelle l'orifice de sortie de la chambre de pompage s'ouvre, les deux parties étant en communication par l'intermédiaire d'une ouverture pratiquée dans la section inférieure (B) de la plaque de séparation.

2. Pompe, selon la revendication 1, dans laquelle l'orifice de sortie servant à décharger du fluide à partir de la chambre de pompage vers la chambre intermédiaire est une ouverture positionnée dans la paroi commune ou située de façon adjacente à cette dernière.

3. Pompe, selon la revendication 1 ou la revendication 2, dans laquelle l'élément de pompage alternatif est un diaphragme.

4. Pompe, selon l'une quelconque des revendications précédentes, dans laquelle l'orifice de sortie de la chambre intermédiaire est formé dans un raccord de décharge qui est attaché au carter.

5. Pompe, selon l'une quelconque des revendications précédentes, dans laquelle l'orifice de sortie de la chambre intermédiaire se rétrécit à partir d'une ouverture relativement grande dans la chambre intermédiaire pour devenir un orifice d'évacuation relativement petit.

6. Pompe, selon l'une quelconque des revendications précédentes, comprenant en outre une tubulure de décharge (24) qui est positionnée à l'intérieur de l'orifice de sortie de la chambre intermédiaire, et est connectée à celui-ci.

7. Pompe, selon la revendication 7, dans laquelle la partie de la chambre intermédiaire contenant le gaz est une partie centrale et la partie dans laquelle l'orifice de sortie de la chambre de pompage s'ouvre, est une partie annulaire externe.

8. Pompe, selon l'une quelconque des revendications précédentes, dans laquelle le carter comprend un premier et un deuxième corps qui sont attachés l'un à l'autre, et qui respectivement définissent, du moins partiellement, la chambre de pompage et la chambre intermédiaire, alors que la plaque de séparation fait partie intégrante du deuxième corps, et aboute contre ladite paroi commune qui fait partie intégrante du premier corps.
